# EUROPÄISCHE PATENTSCHRIFT

(11) **EP 2 377 562 B1**
(45) Veröffentlichungstag und Bekanntmachung des Hinweises auf die Patenterteilung: **04.03.2015**
(21) Anmeldenummer: 11002857.8
(22) Anmeldetag: 06.04.2011
(51) Int. Cl.: A61M 1/00, A61M 3/02, A61M 11/00

(54) **Spülsystem zur Reinigung von Operationswunden**
Rinsing system for cleaning operation wounds
Système de rinçage pour le nettoyage de plaies opératoires

(30) Priorität: 17.04.2010 DE 202010006116 U
(43) Veröffentlichungstag der Anmeldung: 19.10.2011
(73) Patentinhaber: Schwarz, Volker A., 94377 Steinach (DE)
(72) Erfinder: Schwarz, Volker A., 94377 Steinach (DE)
(74) Vertreter: Graf Glück Kritzenberger

(56) Entgegenhaltungen:
- EP-A1- 0 352 984
- US-A- 4 692 140
- US-A- 5 254 117
- US-A- 6 156 004
- US-B1- 6 607 503

## Beschreibung

Die Erfindung betrifft ein Spülsystem zur Reinigung von Operationswunden gemäß dem Oberbegriff des Schutzanspruches 1.

Spülsysteme zur Reinigung von Operationswunden sind hinlänglich aus dem Stand der Technik bekannt. Derartige Spülsysteme werden zur Reinigung von Operationswunden und/oder eines Implantationsortes vor dem Setzen eines Knochenimplantats verwendet und weisen zumindest einen Grundkörper und eine mit diesem verbundenes, mehrteiliges Spülrohr auf. Der Grundkörper wird vorzugsweise an einem Handstück befestigt, über welches eine im Grundkörper vorgesehene Membran mit Druckluft beaufschlagt wird. Mittels der Druckluft wird eine Schwingung der Membran erzeugt, wodurch eine im Grundkörper vorgesehene Pumpenmechanik umfassend mehrere Ventile angeregt wird. Über die Pumpenmechanik wird eine Spül- bzw. Reinigungsflüssigkeit von einer externen Quelle über den Grundkörper einem im Grundkörper aufgenommenen Spülrohr zugeführt und über dieses in die jeweilige Operationswunde eingebracht. Hierzu ist am freien Ende des Spülrohres ein Düsenabschnitt vorgesehen, über welchen eine Streuung des Flüssigkeitsstrahles erzeugt wird.

Die am Reinigungsort vorhandene Flüssigkeit wird über einen Absaugkanal des Spülrohres abgesaugt. Hierzu ist das Spülrohr vorzugsweise zweiteilig ausgebildet, d.h. weist ein Spritzrohr auf, welches in einem Absaugrohr aufgenommen ist. Die Zufuhr der Reinigungsflüssigkeit vom Grundkörper in die Operationswunde erfolgt hierbei über das Spritzrohr, welches zumindest abschnittsweise von dem Absaugrohr umschlossen ist. Das zum Absaugen erforderliche Vakuum wird vorzugsweise über eine externe Vakuumquelle erzeugt, die über einen Absaugschlauch und dem Grundkörper mit dem Absaugrohr verbunden ist. Am freien Ende des Spülrohres bzw. Absaugrohres ist vorzugsweise ein Spritzschutzelement vorgesehen, dass aus einem Verbindungsabschnitt und einem daran anschließenden trichterförmigen Schutzabschnitt besteht, welcher den Saug- und Spritzraum der Operationswunde abdeckt.

Aus der US 4,692,140 sowie der US 6,156,004 sind Spülsysteme zur Reinigung von Operationswunden umfassend einen Grundkörper und ein mehrteiliges Spülrohr bekannt.

Ferner offenbart die US 6,607,503 eine Anordnung zur Follikelpunktion im Rahmen der extrakorporalen Befruchtung mit einem mehrteiligen, doppellumigen Nadelkörper, wobei der Nadelkörper ein inneres Nadelröhrchen aus einem elastischen Kunststoffmaterial aufweist.

Nachteilig sind gemäß dem Stand der Technik ausgebildete Spülrohre, insbesondere das Spritzrohr aus einem steifen und spröden Kunststoffmaterial, vorzugsweise Acrylnitril-Butadien-Styrol hergestellt. Damit ist eine Anpassung des Spülrohres an unterschiedliche Saug- und Reinigungsaufgaben, insbesondere bei schwer zugänglichen Stellen nicht möglich.

Ausgehend hiervon liegt der Erfindung die Aufgabe zugrunde, ein neuartiges Spülsystem zur Reinigung von Operationswunden anzugeben, welche eine verbesserte Anpassung an unterschiedliche Saug- bzw. Reinigungsaufgaben ermöglicht und damit eine einfache Handhabbarkeit gewährleistet. Die Aufgabe wird ausgehend von den Merkmalen des Oberbegriffes des Schutzanspruches 1 durch dessen kennzeichnende Merkmale gelöst.

Der wesentliche Aspekt des neuerungsgemäßen Spülsystems ist darin zu sehen, dass zumindest das Spritzrohr des mehrteiligen Spülrohres aus zumindest zwei elastischen Kunststoffmaterialien hergestellt ist und einen mehrschichtigen Aufbau aufweist. Beispielsweise finden als elastisches Kunststoffmaterial Polyamid und/oder Polyurethan Anwendung. Vorteilhaft gewährleistet das Polyamid eine ausreichende Stabilität des Spritzrohres, während die dünne Außenschicht aus Polyurethan Vorteile für das Verbindung des Spritzrohres mit dem Düsenelement besitzt. Selbst bei extremer Biegebeanspruchung kommt es nicht zum Bruch des Spritzrohres, weil sowohl Polyamid als auch Polyurethan eine deutlich höhere Flexibilität als die herkömmlich verwendeten Materialien wie beispielsweise Acrylnitril-Butadien-Styrol aufweisen. Weiterhin vorteilhaft ist dadurch ein Verbiegen des Spülrohres durch den Operator möglich und somit eine Anpassung an die jeweilige Operationssituation. Weiterhin vorteilhaft ermöglicht die Außenschicht aus Polyurethan ein direktes Aufkleben des Düsenelementes auf das Spritzrohr, wodurch ein zusätzliches Material zur hochfesten Verbindung der Düse und Spülrohr entfällt. Zur Verbindung wird vorzugsweise ein Lösungsmittelklebeverfahren eingesetzt, dass die Oberflächen des Spritzrohres und des damit zu verbindenden Düsenelementes anlöst und damit einen Übergangsbereich ausbildet, indem beide Kunststoffe miteinander verbunden sind. Darüber hinaus werden ggf. vorhandene gelöste Partikel an den Schnittkanten der Spülröhrchen durch das Lösungsmittel angelöst, so dass diese nach dem Vorgang ebenfalls fest anhaften.

Ein weiterer Aspekt der Erfindung ist darin zu sehen, dass das Spritzschutzelement im Übergangsbereich zwischen dem Hülsenabschnitt und dem trichterförmigen Schutzabschnitt einen innen liegenden Anschlag aufweist, an dem das stirnseitige freie Ende des Spülrohres zumindest abschnittsweise anliegt.

In einer bevorzugten Ausführungsform sind die Längen des Spritzrohres und des Absaugrohres unterschiedlich gewählt.

Weiterhin vorteilhaft ist das Spritzschutzelement (7) aus einem elastischen, transparenten Kunststoffmaterial hergestellt und weist eine Shorehärte kleiner 85 Shore A auf. Durch die Verwendung eines derartig weichen Kunststoffmaterials kann besonders vorteilhaft das Spritzschutzelement enger an die vorhandenen Knochen angelegt werden.

Vorteilhafte Weiterbildungen der Erfindung sind Gegenstand der weiteren abhängigen Ansprüche.

Zudem ergeben sich vorteilhafte Weiterbildungen, Vorteile und Anwendungsmöglichkeiten der Erfindung auch aus der nachfolgenden Beschreibung von Ausführungsbeispielen und aus den Figuren. Auch wird der Inhalt der Ansprüche zu einem Bestandteil der Beschreibung gemacht.

Die Erfindung soll nachfolgend anhand von Ausführungsbeispielen im Zusammenhang mit den Figuren näher erläutert werden. Es wird aber ausdrücklich darauf hingewiesen, dass die Erfindung keinesfalls auf die angegebenen Beispiele beschränkt sein soll. Es zeigen
Fig. 1 beispielhaft einen schematischen Längsschnitt durch ein neuerungsgemäßes Spülsystem mit einem Handstück,
Fig. 2 beispielhaft einen schematischen Längsschnitt durch das Spritzschutzelement des neuerungsgemäßes Spülsystems, und
Fig. 3 beispielhaft eine Stirnansicht des Spritzschutzelementes gemäß Fig. 2.

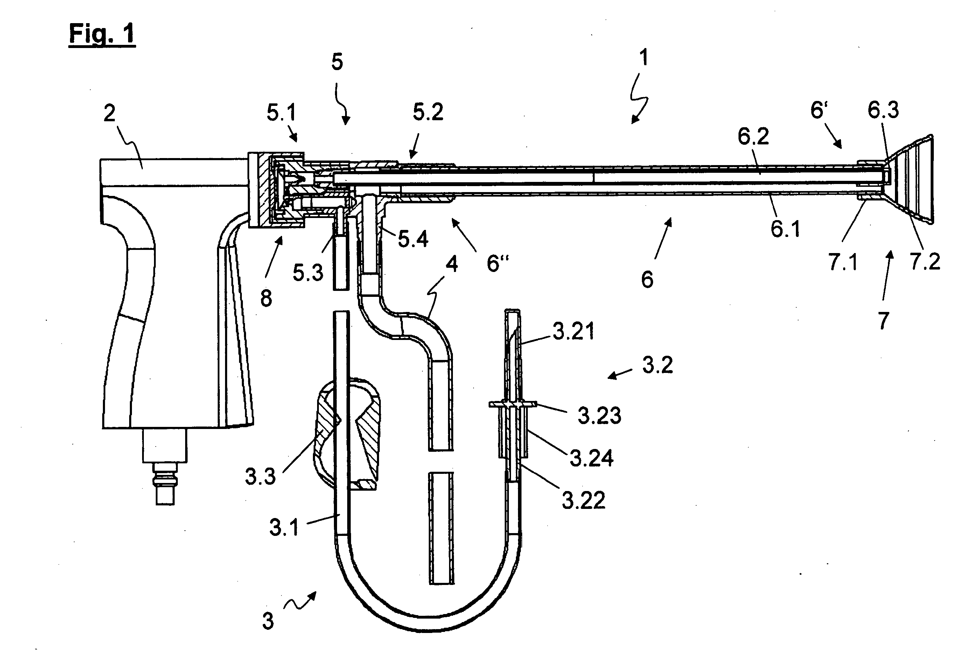

In Figuren 1 ist beispielhaft eine Anordnung zur Reinigung von Operationswunden und/oder Knochenöffnungen dargestellt, welche ein neuerungsgemäßes Spülsystem 1, ein Handstück 2, ein Verbindungsschlauchsystem 3 sowie eine Absaugleitung 4 umfasst.

Bei dem Spülsystem 1 oder auch "Lavage"-System handelt es sich prinzipiell um einen Einmalartikel, welcher nach Gebrauch entsorgt wird. Sämtliche Komponenten des Spülsystems 1 sind aus Kunststoff hergestellt.

Das Spülsystem 1 besteht im Wesentlichen aus einem Grundkörper 5, einem mehrteiligen Spülrohr 6 und einem Spritzschutzelement 7, welches an dem dem Grundkörper 5 gegenüberliegenden freien Ende 6' des Spülrohres 6 vorgesehen ist.

Der Grundkörper 5 weist einen ersten Anschlussbereich 5.1 zur Verbindung mit dem Handstück 2 und einen zweiten Anschlussbereich 5.2 zur Verbindung des weiteren freien Endes 6" des Spülrohres 6 mit dem Grundkörper 5 auf.

Das mehrteilige Spülrohr 6 umfasst zumindest ein Absaugrohr 6.1, in welchem ein Spritzrohr 6.2 zumindest abschnittsweise aufgenommen ist, und zwar ist der Durchmesser des Spritzrohres 6.2 deutlich kleiner als der Durchmesser des Absaugrohres 6.1, so dass sich zwischen dem Absaugrohr 6.1 und dem Spritzrohr 6.2 ein ringförmiger Absaugkanal zur Absaugung der Wundsekretes bzw. der auf die Wunde aufgebrachten Flüssigkeit ausbildet.

Das Spritzrohr 6.2 weist im Vergleich zum Absaugrohr 6.1 darüber hinaus eine unterschiedliche Länge auf, und zwar ist vorzugsweise das Spritzrohr 6.2 länger als das Absaugrohr 6.1 ausgebildet. In einer bevorzugten Ausführungsform ist an den dem Grundkörper 5 zugewandeten freien Ende 6" des Absaugrohres 6.1 und des Spritzrohres 6.2 jeweils ein Außengewinde vorgesehen, welches mit einem jeweils zugehörigen Innengewinde in einer Ausnehmung 5' des Grundkörpers 5 zusammenwirkt. Die Ausnehmung 5' weist vorzugsweise zumindest zwei unterschiedliche Durchmesser aufweisende, im Querschnitt kreisförmige Abschnitte auf, die denen jeweils eines der Innengewinde vorgesehen ist.

Der Grundkörper 5 umfasst ferner einen Zuführungsanschluss 5.3, an dem das Verbindungsschlauchsystem 3 anschließbar ist. Mittels einer im ersten Anschlussbereich 5.1 im Grundkörper 5 vorgesehenen Membranpumpenmechanik 8 wird die über das über den Zuführungsanschluss 5.3 vom Verbindungsschlauchsystem 3 bereitgestellte Reinigungsflüssigkeit in das Spritzrohr 6.2 gefördert. Hierzu weist die Membranpumpenmechanik 8 beispielsweise eine Membran und zwei Ventile auf.

Am freien Ende des Spritzrohres 6.2 ist ein Düsenelement 6.3 angeordnet, welches stirnseitige Öffnungen zur gleichmäßigen Verteilung der Reinigungsflüssigkeit besitzt. Die Öffnungen sind vorzugsweise konzentrisch zur Längsachse des Spritzrohres 6.2 im Düsenelement 6.3 vorgesehen.

Die stirnseitigen Enden des Düsenelementes 6.3 und des Absaugrohres 6.1 sind in einer bevorzugten Ausführungsform näherungsweise in einer gemeinsamen Ebene angeordnet, wobei aufgrund der unterschiedlichen Längen des Absaugrohres 6.1 und des Spritzrohres 6.2 die Befestigung im Grundkörper 5 an entlang der Längsachse des Spülrohres 5 beabstandete Stellen innerhalb der Ausnehmung 5' erfolgt, und zwar sind die hierzu vorgesehenen Innengewinde mit unterschiedlichen Durchmesser über die Ausnehmung 5' voneinander getrennt.

Die Ausnehmung 5' steht im Bereich zwischen den beiden Innengewinden mit einem nach außen abstehenden Absauganschluss 5.4 des Grundkörpers 5 in Wirk- bzw. Fluidverbindung, an dem die Absaugleitung 4 anschließbar ist. Ferner besteht eine Verbindung der Ausnehmung 5' zum Absaugrohr 6.1, so dass die abzusaugende Flüssigkeit vom Behandlungsort über das Absaugrohr 6.1, die Ausnehmung 5' und den Absauganschluss 5.4 in die Absaugleitung 4 geführt werden kann, wobei die Absaugleitung 4 an einen externen Vakuumbehälter angeschlossen ist und somit ein Unterdruck in der Absaugleitung besteht.

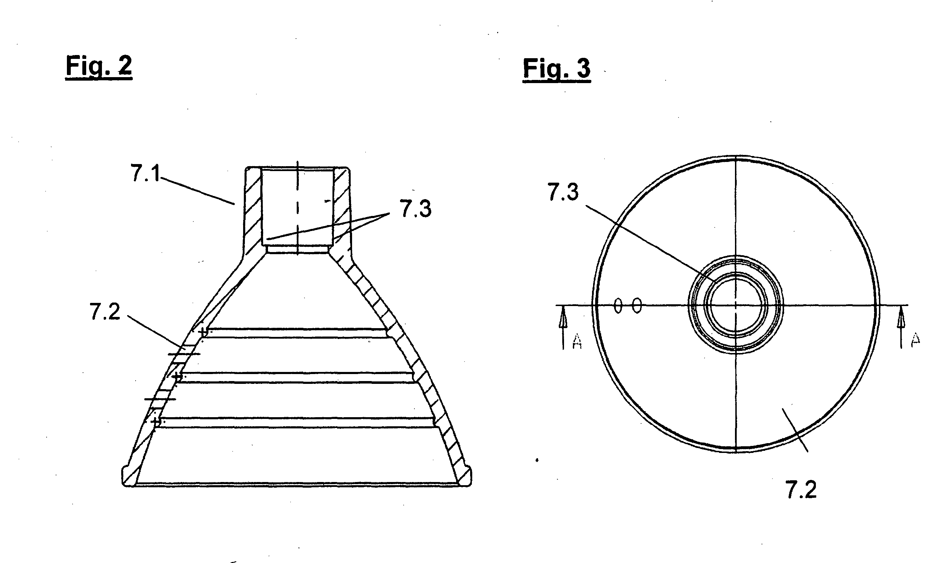

Ferner ist am freien Ende des Absaugrohres 6.1 ein Spritzschutzelement 7 angeordnet, welches einen Hülsenabschnitt 7.1 und einen daran anschließenden trichterförmigen Schutzabschnitt 7.2 umfasst. In den Figuren 2 und 3 sind ein Längsschnitt und eine Stirnansicht des Hülsenabschnittes7 beispielhaft dargestellt. Der Hülsenabschnitt 7.1 dient hierbei zur Befestigung am Absaugrohr 6.1, wobei der trichterförmige Schutzabschnitt 7.2 zu einer verbesserten Reinigung und Absaugung des Behandlungsortes vorgesehen ist, und zwar wird der trichterförmigen Schutzabschnitt 7.2 des Spritzschutzelementes 7 auf die Operationswunde aufgebracht.

Über das Handstück 2 wird die Membranpumpenmechanik 8, insbesondere die darin vorgesehene Membran mit Druck beaufschlagt, wodurch ein mittels des Verbindungsschlauchsystems 3 zugeführte Reinigungsflüssigkeit in das Spritzrohr 6.2 gefördert und über das Düsenelement 6.3 dem Behandlungsort zugeführt wird.

Neuerungsgemäß ist zumindest das Spritzrohr 6.2 aus zwei elastischen Kunststoffmaterialien hergestellt, welche nicht transparent, halbtransparent oder transparent ausgebildet sein können. Auch das Absaugrohr 6.1 ist zumindest abschnittsweise aus einem transparenten Kunststoffmaterial gefertigt. Weiterhin weist zumindest das Spritzrohr 6.2 einen mehrschichtigen Aufbau auf, beispielsweise eine innere Schicht aus Polyurethan und eine äußere Schicht aus Polyamid. Hierbei ist die innere Schicht aus Polyurethan deutlich dicker als die äußere Schicht aus Polyamid. Beispielsweise weist die innere Schicht eine Dicke zwischen 0,8 und 0,9 mm und die äußere Schicht eine Dicke von 0,1 bis 0,2 mm auf.

Weiterhin vorteilhaft ist im Übergangsbereich zwischen dem Hülsenabschnitt 7.1 und dem trichterförmigen Schutzabschnitt 7.2 des Spritzschutzelementes 7 zumindest ein innerer Anschlag 7.3 vorgesehen, an dem das stirnseitige freie Ende des Absaugrohres 6.1 zumindest abschnittsweise anliegt.

Der innen liegende Anschlag 7.3 kann beispielsweise ringförmig ausgebildet sein.

Hierzu ist der Durchmesser der Durchlassöffnung des trichterförmigen Schutzabschnittes 7.2 im Vergleich zum Außendurchmesser des Absaugrohres 6.1 bzw. des Innendurchmessers des Hülsenabschnittes 7.1 reduziert. Damit liegt das freie Ende des Absaugrohres 6.1 an dem ringförmigen Anschlag 7.3 an. Hierdurch wird effektiv ein zu weites Einschieben des Spülrohres 6 bzw. Absaugrohres 6.1 in den Hülsenabschnitt 7.1, und zwar über den Hülsenabschnitt hinaus in den trichterförmigen Schutzabschnitt 7.2, vermieden.

Besonders vorteilhaft ist das Spritzschutzelement 7 ebenfalls aus einem elastischen, besonders weichen Kunststoffmaterial hergestellt, welches vorzugsweise halttransparent oder transparent ausgebildet ist und/oder eine Shorehärte kleiner 85 Shore A aufweist. Durch die dadurch erhöhte Flexibilität des trichterförmigen Schutzabschnittes 7.2 ein verbessertes Umschließen des Behandlungsortes.

Weiterhin vorteilhaft umfasst das Verbindungsschlauchsystem 3 ein Schlauchelement 3.1, eine Einstecheinheit 3.2 sowie eine Schlauchklemme 3.3. Mittels der Einstecheinheit 3.2 ist das Schlauchsystem beispielsweise an eine Infusionsflasche anschließbar, wobei die Einstecheinheit 3.2 eine verstärkte Anstechspitze 3.21 und einen Schlauchanschluss 3.22 aufweist, die über ein Anschlagflanschelement 3.23 verbunden sind. Neuerungsgemäß verfügt die Anstechspitze 3.21 über eine im Vergleich zu bekannten Anstechspitzen über eine erhöhte Wandstärke. Beispielsweise beträgt die Wandstärke der Anstechspitze 3.21 zwischen 1 und 1,1 mm. Die an dem der Anstechspitze 3.21 gegenüberliegenden Ende vorgesehene Schlauchanschluss 3.22 ist in einer bevorzugten Ausführungsform verstärkt ausgebildet, und zwar ist im Bereich des Schlauchanschluss 3.22 an der Außenseite der Anstecheinheit 3.2 eine Verstärkung zur Verbesserung der Griffigkeit vorgesehen. Die Verstärkung ist beispielsweise durch einen den Schlauchanschluss 3.22 zumindest teilweise hülsenartigen umgebenden Wandabschnitt 3.24 gebildet, der beispielsweise einen kreisförmigen, ovalen, rechteckförmigen oder quadratischen Querschnitt aufweist und sich ausgehend vom Anschlagflanschelement 3.2 in Richtung des Schlauchanschlusses 3.22 erstreckt.

Die Schlauchklemme 3.3 ist zur Unterbindung der Zufuhr von weiterer Reinigungsflüssigkeit über das Verbindungsschlauchsystem 3 an das Spülsystem 1 vorgesehen. Hierzu weist die Schlauchklemme 3.3 im Wesentlichen zwei Betriebszustände auf, und zwar "geöffnet" und "verschlossen", wobei im Betriebszustand "geöffnet" der Durchfluss der Reinigungsflüssigkeit durch das Schlauchelement 3.1 freigegeben ist und im Betriebszustand "verschlossen" ein Verschluss des Schlauchelementes 3.1 mittels der Schlauchklemme 3.3 erfolgt. Aufgrund der lediglich zwei Betriebszustände wird ein fehlerbehaftetes Einstellen eines weiteren, eine Zwischenposition betreffenden Betriebzustandes vermieden.

Die Erfindung wurde voranstehend an einem Ausführungsbeispiel beschrieben. Es versteht sich, dass zahlreiche Modifikationen und Änderungen der Erfindung möglich sind, ohne dass hierdurch der Erfindungsgedanke verlassen wird, der durch die folgenden Ansprüche definiert ist.

### Bezugszeichenliste

- 1: Spülsystem
- 2: Handstück
- 3: Verbindungsschlauchsystem
- 3.1: Schlauchelement
- 3.2: Einstecheinheit
- 3.21: Einsteckspitze
- 3.22: Schlauchanschluss
- 3.23: Anschlagflanschelement
- 3.24: Wandabschnitt
- 3.3: Schlauchklemme
- 4: Absaugleitung
- 5: Grundkörper
- 5': Ausnehmung
- 5.1: erster Anschlussbereich
- 5.2: zweiter Anschlussbereich
- 5.3: Zuführungsanschluss
- 5.4: Absauganschluss
- 6: Spülrohr
- 6.1: Absaugrohr
- 6.2: Spritzrohr
- 6.3: Düsenelement
- 6': freies Ende
- 6": freies Ende
- 7: Spritzschutzelement
- 7.1: Hülsenabschnitt
- 7.2: trichterförmiger Schutzabschnitt
- 7.3: Anschlag
- 8: Membranpumpenmechanik

## Patentansprüche

1. Spülsystem zur Reinigung von Operationswunden umfassend einen Grundkörper (5) und zumindest ein mehrteiliges Spülrohr (6), bei dem der Grundkörper (5) einen ersten Anschlussbereich (5.1) zum Anschluss des mehrteiligen Spülrohres (6) und einen zweiten Anschlussbereich (5.2) zum Anschluss eines Handstückes (2) aufweist, bei dem das mehrteilige Spülrohr (6) zumindest ein Absaugrohr (6.1) und ein darin zumindest abschnittsweise aufgenommenes Spritzrohr (6.2) aufweist und bei dem an dem dem Grundkörper (5) gegenüber liegenden freien Ende (6') des Spritzrohres (6.2) ein Düsenelement (6.3) vorgesehen ist, **dadurch gekennzeichnet, dass** zumindest das Spritzrohr (6.2) des mehrteiligen Spülrohres (6) aus zumindest zwei elastischen Kunststoffmaterialien hergestellt ist und einen mehrschichtigen Aufbau aufweist.

2. Spülsystem nach Anspruch 1, **dadurch gekennzeichnet, dass** als elastisches Kunststoffmaterial Polyamid und/oder Polyurethan Anwendung finden.

3. Spülsystem nach Anspruch 1, **dadurch gekennzeichnet, dass** eine innere Schicht aus Polyamid und eine äußere Schicht aus Polyurethan vorgesehen ist, wobei die Dicke der inneren Schicht vorzugsweise zwischen 0,8 und 0,9 mm und die Dicke der äußeren Schicht vorzugsweise zwischen 0,1 und 0,2 mm gewählt ist.

4. Spülsystem nach einem der Ansprüche 1 bis 3, **dadurch gekennzeichnet, dass** an dem dem Grundkörper (5) gegenüber liegenden freien Ende (6') des Absaugrohres (6.2) ein Spritzschutzelement (7) angeordnet ist.

5. Spülsystem nach Anspruch 4, **dadurch gekennzeichnet, dass** das Spritzschutzelement (7) einen Hülsenabschnitt (7.1) und einen daran anschließenden trichterförmiges Schutzabschnitt (7.2) aufweist, wobei im Übergangsbereich zwischen dem Hülsenabschnitt (7.1) und dem trichterförmigen Schutzabschnitt (7.2) zumindest ein innerer Anschlag (7.3) vorgesehen ist.

6. Spülsystem nach Anspruch 5, **dadurch gekennzeichnet, dass** der innere Anschlag (7.3) einen Anschlag für das im Hülsenabschnitt (7.1) aufgenommene stirnseitige, freie Ende des Absaugrohres (6.2) bildet.

7. Spülsystem nach Anspruch 5 oder 6, **dadurch gekennzeichnet, dass** der innen liegende Anschlag (7.3) ringförmig ausgebildet ist.

8. Spülsystem nach einem der Ansprüche 5 bis 7, **dadurch gekennzeichnet, dass** zur Ausbildung des inneren Anschlags (7.3) der Durchmesser der Durchlassöffnung des trichterförmigen Schutzabschnittes (7.2) im Vergleich zum Außendurchmesser des Absaugrohres (6.1) bzw. des Innendurchmessers des Hülsenabschnittes (7.1) reduziert ist.

9. Spülsystem nach einem der Ansprüche 1 bis 8, **dadurch gekennzeichnet, dass** das Absaugrohr (6.1) und das Spritzrohr (6.2) unterschiedliche Längen aufweisen und/oder dass an dem dem Grundkörper (5) gegenüber liegenden freien Ende (6') des Spritzrohres (6.2) ein Düsenelement (6.3) angeordnet ist, wobei das Düsenelement (6.3) mit der äußeren Kunststoffschicht des Spritzrohres (6.2) fest verbunden, vorzugsweise verklebt ist.

10. Spülsystem nach einem der Ansprüche 4 bis 9, **dadurch gekennzeichnet, dass** das Spritzschutzelement (7) aus einem elastischen Kunststoffmaterial hergestellt ist, welches vorzugsweise halbtransparent oder transparent ausgebildet ist und/oder eine Shorehärte kleiner 85 Shore A aufweist.

11. Spülsystem nach einem der vorhergehenden Ansprüche, **dadurch gekennzeichnet, dass** der Grundkörper (5) einen Zuführungsanschluss (5.3) zum Anschluss eines Verbindungsschlauchsystems (3) und einen Absauganschluss zum Anschluss einer Absaugleitung (4) aufweist.

12. Anordnung bestehend aus einem Spülsystem (1) nach einem der vorhergehenden Ansprüche, einem Verbindungsschlauchsystem (3) und einer Absaugleitung (4), **dadurch gekennzeichnet, dass** das Verbindungsschlauchsystem (3) zumindest ein Schlauchelement (3.1), eine Einstecheinheit (3.2) und eine Schlauchklemme (3.3) umfasst, wobei die Einsteckeinheit (3.2) eine verstärkte Einsteckspitze (3.21) aufweist.

13. Anordnung nach Anspruch 12, **dadurch gekennzeichnet, dass** die verstärkte Einsteckspitze (3.21) eine Wandstärke zwischen 1 und 1,1 mm aufweist.

14. Anordnung nach Anspruch 12 oder 13, **dadurch gekennzeichnet, dass** die Einstecheinheit (3.2) neben der verstärkten Anstechspitze (3.21) einen verstärkten Schlauchanschluss (3.22) aufweist, der vorzugsweise durch einen den Schlauchanschluss (3.22) zumindest teilweise hülsenartig umgebenden Wandabschnitt (3.24) gebildet ist und/oder dass die Schlauchklemme (3.3) zwei Betriebzustände aufweist, und zwar einen geöffneten und einen geschlossenen Zustand.

## Claims

1. A rinsing system for cleaning operation wounds, comprising a main body (5) and at least a multi-part rinsing tube (6), the main body (5) having a first connection region (5.1) for connection of the multi-part rinsing tube (6) and a second connection region (5.2) for connection of a hand-piece (2), the multi-part rinsing tube (6) having at least a suction tube (6.1) and an injection tube (6.2) received therein at least in portions, and a nozzle element (6.3) being provided at the free end (6') of the injection tube (6.2) opposite the main body (5), **characterised in that** at least the injection tube (6.2) of the multi-part rinsing tube (6) is produced from at least two elastic plastic materials and has a multi-layer structure.

2. The rinsing system according to claim 1, **characterised in that** polyamide and/or polyurethane are used as elastic plastic material.

3. The rinsing system according to claim 1, **characterised in that** an inner layer made of polyamide and an outer layer made of polyurethane is provided, wherein the thickness of the inner layer is preferably selected between 0.8 and 0.9 mm and the thickness of the outer layer is preferably selected between 0.1 and 0.2 mm.

4. The rinsing system according to one of claims 1 to 3, **characterised in that** a splash guard element (7) is arranged at the free end (6') of the suction tube (6.2) opposite the main body (5).

5. The rinsing system according to Claim 4, **characterised in that** the splash guard element (7) has a sleeve portion (7.1) and a funnel-shaped protection portion (7.2) connected thereto, wherein at least one inner stopper (7.3) is provided in the transition region between the sleeve portion (7.1) and the funnel-shaped protection portion (7.2).

6. The rinsing system according to claim 5, **characterised in that** the inner stopper (7.3) forms a stopper for the front free end of the suction tube (6.2) received in the sleeve portion (7.1).

7. The rinsing system according to claim 5 or 6, **characterised in that** the inner stopper (7.3) is formed annularly.

8. The rinsing system according to one of claims 5 to 7, **characterised in that**, in order to form the inner stopper (7.3), the diameter of the through-opening of the funnel-shaped protection portion (7.2) is reduced as compared to the outer diameter of the suction tube (6.1) or the inner diameter of the sleeve portion (7.1).

9. The rinsing system according to one of claims 1 to 8, **characterised in that** the suction tube (6.1) and the injection tube (6.2) have different lengths, and/or **in that** a nozzle element (6.3) is arranged at the free end (6') of the injection tube (6.2) opposite the main body (5), wherein the nozzle element (6.3) is fixedly connected, preferably adhered, to the outer plastic layer of the injection tube (6.2).

10. The rinsing system according to one of claims 4 to 9, **characterised in that** the splash guard element (7) is produced from an elastic plastic material that is preferably semi-transparent or transparent and/or has a Shore hardness less than 85 Shore A.

11. The rinsing system according to one of the preceding claims, **characterised in that** the main body (5) has a supply connector (5.3) for connection of a connecting hose system (3) and a suction connector for connection of a suction line (4).

12. An arrangement consisting of a rinsing system (1) according to one of the preceding claims, a connecting hose system (3) and a suction line (4), **characterised in that** the connecting hose system (3) comprises at least one hose element (3.1), a piercing unit (3.2) and a hose clamp (3.3), wherein the piercing unit (3.2) has a reinforced piercing tip (3.21).

13. The arrangement according to Claim 12, **characterised in that** the reinforced piercing tip (3.21) has a wall thickness between 1 and 1.1 mm.

14. The arrangement according to Claim 12 or 13, **characterised in that** the piercing unit (3.2), besides the reinforced piercing tip (3.21), has a reinforced hose connector (3.22), which is preferably formed by a wall portion (3.24) surrounding the hose connector (3.22) in a sleeve-like manner at least in part, and/or **in that** the hose clamp (3.3) has two operating states, more specifically an open and a closed state.

## Revendications

1. Système de rinçage pour le nettoyage de plaies opératoires, comprenant un corps de base (5) et au moins un tuyau de rinçage en plusieurs pièces (6), dans lequel le corps de base (5) présente une première zone de raccordement (5.1) pour le raccordement du tuyau de rinçage en plusieurs pièces (6) et une seconde zone de raccordement (5.1) pour le raccordement d'une pièce manuelle (2), dans lequel le tuyau de rinçage en plusieurs pièces (6) présente au moins un tuyau d'aspiration (6.1) et un tuyau d'injection (6.2) reçu dedans au moins par sections et dans lequel un élément à buse (6.3) est prévu à l'extrémité (6') opposée au corps de base (5) du tuyau d'injection (6.2), **caractérisé en ce qu'**au moins le tuyau d'injection (6.2) du tuyau de rinçage en plusieurs pièces (6) est réalisé à partir d'au moins deux matières plastiques élastiques et présente une structure multicouche.

2. Système de rinçage selon la revendication 1, **caractérisé en ce qu'**on utilise comme matière plastique élastique du polyamide et/ou du polyuréthane.

3. Système de rinçage selon la revendication 1, **caractérisé en ce qu'**une couche intérieure en polyamide et une couche extérieure en polyuréthane sont prévues, l'épaisseur de la couche intérieure étant sélectionnée de préférence entre 0,8 et 0,9 mm et l'épaisseur de la couche extérieure préférence entre 0,1 et 0,2 mm.

4. Système de rinçage selon une des revendications 1 à 3, **caractérisé en ce que**, à l'extrémité libre (6') opposée au corps de base (5) du tuyau d'aspiration (6.1), un élément de protection contre les éclaboussures (7) est disposé.

5. Système de rinçage selon la revendication 4, **caractérisé en ce que** l'élément de protection contre les éclaboussures (7) présente une section en manchon (7.1) et une section de protection en forme d'entonnoir (7.2) s'y raccordant, au moins une butée intérieure (7.3) étant prévue dans la zone de transition entre la section en manchon (7.1) et la section de protection en forme d'entonnoir (7.2).

6. Système de rinçage selon la revendication 5, **caractérisé en ce que** la butée intérieure (7.3) constitue une butée pour l'extrémité libre avant du tuyau d'aspiration (6.2) reçue dans la section en manchon (7.1).

7. Système de rinçage selon la revendication 5 ou 6, **caractérisé en ce que** la butée intérieure (7.3) a une conformation annulaire.

8. Système de rinçage selon une des revendications 5 à 7, **caractérisé en ce que**, pour former la butée intérieure (7.3), le diamètre de l'orifice de passage de la section de protection en forme d'entonnoir (7.2) est réduit comparativement au diamètre extérieur du tuyau d'aspiration (6.1) ou au diamètre extérieur de la section en manchon (7.1).

9. Système de rinçage selon une des revendications 1 à 8, **caractérisé en ce que** le tuyau d'aspiration (6.1) et le tuyau d'injection (6.2) présentent des longueurs différentes et/ou qu'un élément à buse (6.3) est disposé à l'extrémité libre (6') opposée au corps de base (5) du tuyau d'injection (6.2), l'élément à buse (6.3) étant relié fixement, de préférence collé, à la couche de plastique extérieure du tuyau d'injection (6.2).

10. Système de rinçage selon une des revendications 4 à 9, **caractérisé en ce que** l'élément de protection contre les éclaboussures (7) est réalisé à partir d'une matière plastique élastique qui est de préférence de type semi-transparent ou transparent et/ou présente une dureté Shore inférieure à 85 Shore A.

11. Système de rinçage selon une des revendications précédentes, **caractérisé en ce que** le corps de base (5) présente un raccord d'acheminement (5.3) pour le raccordement d'un système de tuyau de connexion (3) et un raccord d'aspiration pour le raccordement d'une conduite d'aspiration (4).

12. Dispositif consistant en un système de rinçage (1) selon une des revendications précédentes, un système de tuyau de connexion (3) et une conduite d'aspiration (4), **caractérisé en ce que** le système de tuyau de connexion (3) comprend au moins un élément tubulaire (3.1), une unité de piquage (3.2) et une pince à tuyau (3.3), l'unité de piquage (3.2) présentant une pointe de piquage renforcée (3.21).

13. Dispositif selon la revendication 12, **caractérisé en ce que** la pointe de piquage renforcée (3.21) a une épaisseur de paroi comprise entre 1 et 1,1 mm.

14. Dispositif selon la revendication 12 ou 13, **caractérisé en ce que** l'unité de piquage (3.2), outre la pointe de piquage renforcée (3.21), présente un raccord de tuyau renforcé (3.22) qui est constitué de préférence par une section de paroi (3.24) entourant au moins partiellement à la manière d'un manchon le raccord de tuyau (3.22) et/ou que la pince à tuyau (3.3) présente deux positions de fonctionnement, à savoir une position ouverte et une position fermée.
